# EUROPEAN PATENT SPECIFICATION

(11) **EP 2 403 458 B1**
(45) Date of publication and mention of the grant of the patent: **02.10.2013**
(21) Application number: 10748874.4
(22) Date of filing: 02.03.2010
(51) Int. Cl.: A61F 13/15, A61F 13/49

(54) **FOLDING APPARATUS AND METHOD OF MANUFACTURING ABSORBENT ARTICLE**
FALTVORRICHTUNG UND VERFAHREN ZUR HERSTELLUNG EINES SAUGFÄHIGEN ARTIKELS
APPAREIL DE PLIAGE ET PROCÉDÉ DE FABRICATION D'UN ARTICLE ABSORBANT

(30) Priority: 02.03.2009 JP 2009048485; 26.02.2010 JP 2010041995
(43) Date of publication of application: 11.01.2012
(73) Proprietor: Unicharm Corporation, Ehime 799-0111 (JP)
(72) Inventor: YAMAMOTO, Hiroki, Kanonji-shi Kagawa 769-1602 (JP)
(74) Representative: Syrtsova, Ekaterina
(86) International application number: PCT/JP2010/053740
(87) International publication number: WO 2010/101283

(56) References cited:
- EP-A1- 1 607 357
- EP-A2- 0 388 042
- EP-A2- 1 504 738
- JP-A- 6 247 622
- JP-A- 2001 145 993
- JP-A- 2005 046 246
- JP-A- 2005 298 164
- JP-A- 2008 023 859
- JP-U- 58 158 057
- US-A1- 2003 176 266

## Description

### Technical Field

The present disclosure relates to a folding apparatus for folding a continuous web into two along a folding line extending in a conveyance direction of the web by bringing one side edge of the web toward the opposite side edge thereof, and a method of manufacturing an absorbent article.

### Background Art

An absorbent article, such as a pants-type disposable diaper, includes: a front waistline portion to be fitted to the front waist of a wearer; a back waistline portion to be fitted to the back waist of the wearer; and a crotch portion to be fitted to the crotch of the wearer. Here, leg-surrounding openings (leg holes, for example) into which the legs of the wearer are to be inserted are formed at both sides of the crotch portion.

Japanese Patent Application Publication No. 2005-46246 discloses a method of manufacturing such an absorbent article. The method includes: a forming step of forming leg-surrounding openings in a continuous web to form an absorbent article including a front waistline portion, a back waistline portion, a crotch portion and the like; and a folding step of causing a folding apparatus to fold the web into two along a folding line extending in a conveyance direction of the web by bringing one side edge of the web toward the opposite side edge thereof.

Specifically, the folding apparatus at least includes a large-diameter roller, a folding guide bar, and multiple guide rollers. The large-diameter roller presses a reference region of the web extending from the folding line to the opposite side edge. The folding guide bar supports the web at the folding line. The guide rollers guide a folded region of the web toward the reference region, the folded region extending from the folding line to the one side edge.

The inventors have discovered that, in the known folding apparatus mentioned above, when the folding apparatus folds the web into two by bringing one side edge of the web toward the opposite side edge thereof, the folding guide bar is in contact with a portion of the web to form the crotch portion. With this contact, the portion to form the crotch portion is conveyed behind portions of the web to form the front waistline portion and the back waistline portion, which sometimes causes the web to be folded with the one side edge placed on the opposite side edge but with the portion to form the crotch portion twisted.

Such a twist adversely affects a part ranging from the front waistline portion to the crotch portion, and a part ranging from the back waistline portion to the crotch portion. To be more specific, the twist displaces the front waistline portion and the back waistline portion from each other in their joint portions when the folding step described above is finished, and accordingly distorts and deforms the entire absorbent article. This may consequently degrades the appearance of the absorbent article significantly and result in a defective absorbent article.

It is desirable to provide a folding apparatus and a method of manufacturing an absorbent article which are capable of more reliably inhibiting a defective absorbent article from being manufactured due to a twist of a crotch portion including a folding line, where the folding apparatus folds a web to form an absorbent article into two by bringing one side edge of the web toward the opposite side edge thereof.

### Citation List

### Patent Literature

PTL 1: Japanese Patent Application Publication No. 2005-46246 (pages 6 and 7, and Figs. 3 and 4)

### Summary

To solve the above-described problem, the present invention has the following aspects. An aspect of the present invention provides a folding apparatus by which a continuous web to form an absorbent article is folded into two along a folding line extending in a conveyance direction of the web by bringing one side edge of the web toward an opposite side edge thereof. The folding apparatus includes a pressing mechanism configured to press a first region extending from the folding line to the opposite side edge, a supporting mechanism configured to support the web at the folding line, and a guide mechanism configured to guide a second region toward the first region, the second region extending from the folding line to the one side edge. The supporting mechanism includes a slide portion configured to allow the web to slide thereon. The slide portion has a friction coefficient of not more than 0.4.

The aspect of the present invention can provide a folding apparatus and a method of manufacturing an absorbent article which are capable of more reliably inhibiting a defective absorbent article from being manufactured due to the twist of a crotch portion including a folding position line, in a case where the folding apparatus folds a web to form an absorbent article into two by bringing one side edge of the web toward the opposite side edge thereof.

The apparatus and method in accordance with the invention are defined in the appended claims.

### Brief Description of Drawings

[Fig. 1] Fig. 1 is a partially cutaway, perspective view of an absorbent article according to one or more embodiments.
[Fig. 2] Fig. 2 is a diagram for explaining a relevant part of a method of manufacturing absorbent articles according to one or more embodiments.
[Fig. 3] Fig. 3 is a side view of a folding apparatus according to a first embodiment.
[Fig. 4] Fig. 4 is a perspective view of the folding apparatus of FIG. 3.
[Fig. 5] Fig. 5 is an end view as seen in the direction of an arrow A of Fig. 4.
[Fig. 6] Parts (a) and (b) of Fig. 6 are perspective views of various configuration s of a folding guide bar according to a second embodiment.
[Fig. 7] Fig. 7 is a perspective view of a folding guide bar according to a third embodiment.

### Detailed Description

Hereinafter, a folding apparatus and a method of manufacturing absorbent articles according to one or more embodiments of the present invention will be described with reference to the accompanying drawings. Specifically, first to third embodiments and other embodiments will be described.

Note that, in the following description of the drawings, same or similar reference signs denote same or similar elements and portions. In addition, it should be noted that the drawings are schematic and are not to scale unless otherwise specified.

Therefore, specific dimensions and the like should be determined in consideration of the following description. Moreover, the drawings do not necessarily reflect the real-life dimensional relationships and ratios of components.

### First Embodiment

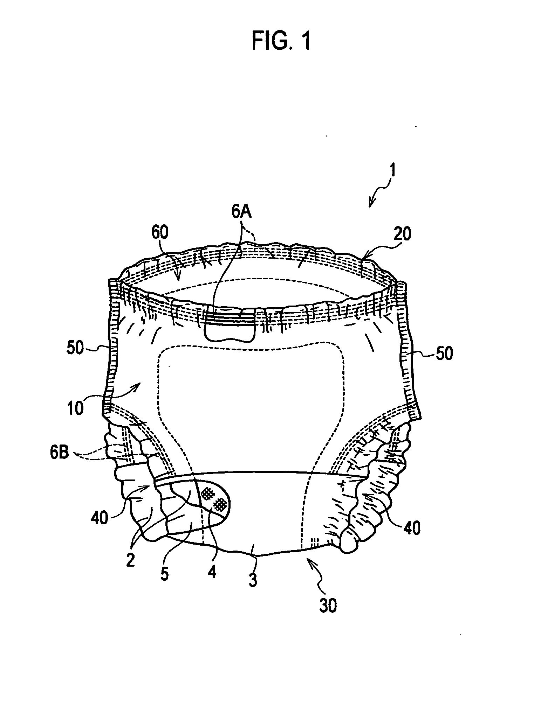

Firstly, a configuration of an absorbent article 1 according to one or more embodiments will be explained with reference to Fig. 1 which is a partially cutaway perspective view of the absorbent article 1. In the particularly illustrated embodiment, the absorbent article 1 is a pants-type disposable diaper for adults.

As shown in Fig. 1, the absorbent article 1 mainly includes a top sheet 2, a back sheet 3, an absorber 4 and a waterproof sheet 5.

The top sheet 2 is adapted to come into contact with the skin of a person to wear the absorbent article 1 (hereinafter, "wearer"). The top sheet 2 is made of a liquid permeable sheet such as a nonwoven fabric or perforated plastic film. The back sheet 3 is provided outside the top sheet 2 (on a side facing away from the wearer). The back sheet 3 is made of a nonwoven fabric or the like.

The absorber 4 is provided between the top sheet 2 and the back sheet 3, for absorbing excretion discharged from the wearer. The absorber 4 is made of a mixture of ground pulp and superabsorbent polymer particles, or the like. The waterproof sheet 5 is provided between the back sheet 3 and the absorber 4, for blocking the permeation of the excretion from the wearer to the outside of the absorbent article 1. The waterproof sheet 5 is made of a liquid impermeable sheet.

The absorbent article 1 thus configured includes, in combination, a front waistline portion 10 to be fitted to the front waist of a wearer, a back waistline portion 20 to be fitted to the back waist of the wearer, and a crotch portion 30 to be fitted to the crotch of the wearer. Incidentally, leg-surrounding openings 40 into which the legs of the wearer are inserted are formed at both sides of the crotch portion 30.

The front waistline portion 10 and the back waistline portion 20 are joined together by joint portions 50, and thereby form a waist opening 60 to be fit around the body of the wearer. A waist gather 6A made of a stretchable rubber strand or the like is provided to an entire peripheral edge of the front waistline portion 10 and the back waistline portion 20.

For example, to make the front waistline portion 10 and the back waistline portion 20 stretchable in the cross direction perpendicular to the front-to-back direction from the front waistline portion 10 toward the back waistline portion 20, the front waistline portion 10 and the back waistline portion 20 may be provided with the waist gather 6A, or may themselves be made of a stretchable sheet.

The crotch portion 30 is provided between the front waistline portion 10 and the back waistline portion 20. Leg gathers 6B made of stretchable rubber strands or the like are provided on both sides of the crotch portion 30.

For example, to make the crotch portion 30 stretchable in the leg-encircling direction of the absorbent article 1, the crotch portion 30 may be provided with the leg gathers 6B, or may itself be made of a stretchable sheet.

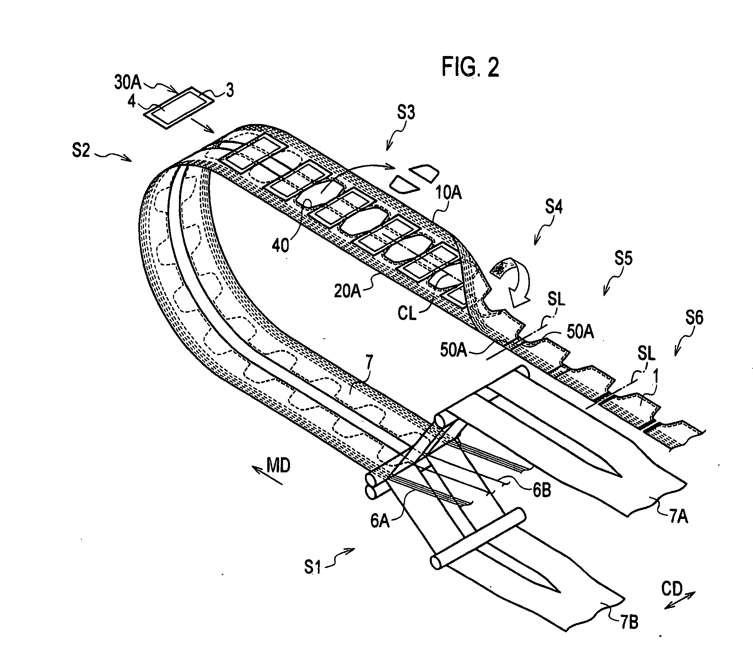

Secondly, a method of manufacturing absorbent articles according to one or more embodiments will be explained with reference to Fig. 2 which is a diagram for explaining a relevant part of the absorbent article manufacturing method.

As shown in Fig. 2, the method of manufacturing absorbent articles includes at least a waistline forming step S1, an absorber transferring step S2, a leg-surrounding opening forming step S3, a folding step S4, a joining step S5 and a cutting step S6.

In the waistline forming step S1, gathers (a waist gather 6A and/or a leg gather 6B) are placed between a web 7A and a web 7B, and thereby a web 7 prepared to form the front waistline portion 10 and the back waistline portion 20 is formed.

Note that the web 7 (webs 7A, 7B) during conveyance is stretchable in a cross direction CD (width direction) perpendicular to a conveyance direction MD (machine direction) of the web 7. In addition, the web 7 is asymmetrical with respect to a center line CL that bisects a width in the cross direction CD of the web 7 and extends in the conveyance direction MD of the web 7.

In the absorber transferring step S2, a crotch portion member 30A to form the crotch portion 30 is transferred onto the web 7, more specifically, between the front waistline portion 10 and the back waistline portion 20 after the waistline forming step S1. Here, the crotch portion member 30A includes the back sheet 3 and the absorber 4.

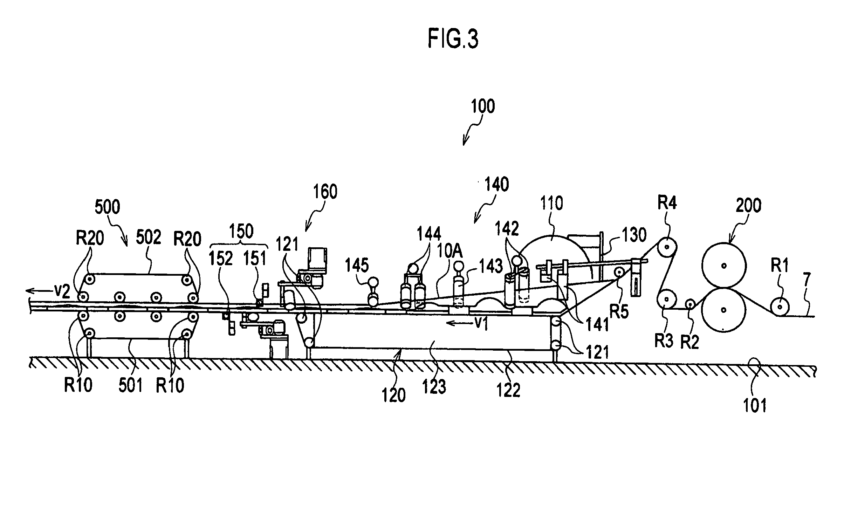

In the leg-surrounding opening forming step S3, a pair of cutting rollers 200 (see Fig. 3) form the leg-surrounding openings 40 (so-called leg holes) by cutting the web 7 (webs 7A, 7B) after the absorber transferring step S2. Here, the formation of the leg-surrounding openings 40 does not necessarily involve cutting only the web 7A and the web 7B, but may involve cutting, together with the web 7A and the web 7B, the back sheet 3 constituting the crotch portion member 30A.

Note that the absorber transferring step S2 and the leg-surrounding forming step S3 may be performed in the reverse order.

In the folding step S4, a folding apparatus 100 to be described later folds the web 7 into two along a folding line extending in the conveyance direction MD of the web 7 by bringing one side edge (first side edge 10A) of the front waistline portion 10 toward the opposite side edge (second side edge 20A) of the back waistline portion 20, after the leg-surrounding opening forming step S3.

Note that, in this particularly illustrated embodiment, the folding line is the center line CL. However, the folding line is not necessarily the center line CL, and may be shifted from the center line CL toward either of the first side edge 10A and the second side edge 20A.

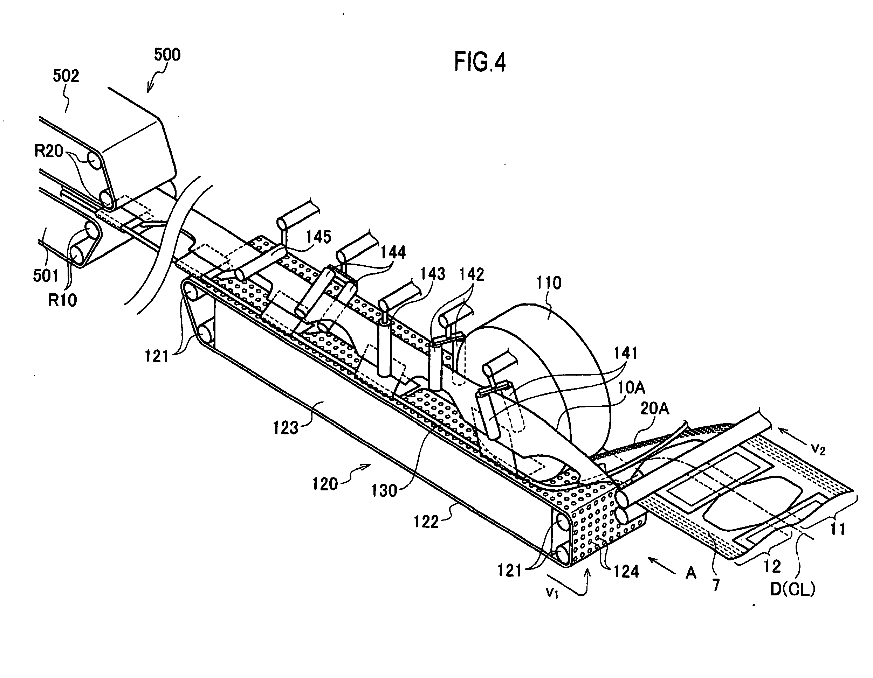

Here, the web 7 having been subjected to the folding step S4 is conveyed while being held by a pair of holding belt conveyors 500 (see Figs. 3 and 4) to be described later.

In the joining step S5, the folded parts of the web 7 are joined together in joint regions 50A, through ultrasonic treatment or heat treatment after the folding step S4. The joint region 50A extends, in the conveyance direction MD, across an imaginary line SL that indicates a to-be-cut position and extends in the cross direction CD of the web 7.

In the cutting step S6, the web 7 joined in the joint regions 50A is cut along the imaginary lines SL after the joining step S5. Thereby, the absorbent article 1 is manufactured.

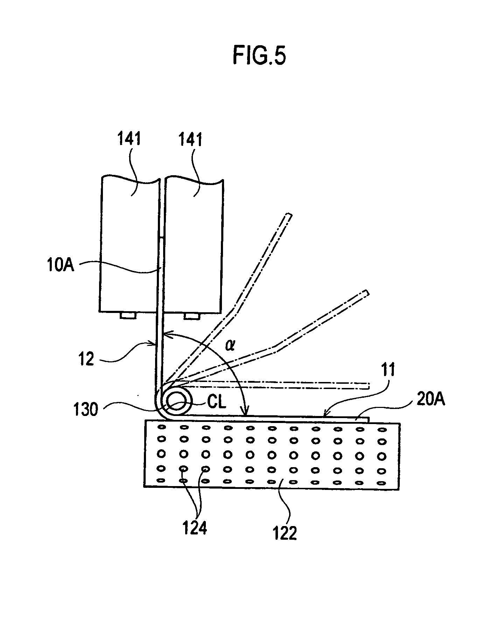

Next, a configuration of the folding apparatus 100 used in the folding step S4 will be explained with reference to Figs. 3-5. Fig. 3 is a side view of the folding apparatus 100 according to the first embodiment. Fig. 4 is a perspective view of the folding apparatus 100 of FIG. 3. Fig. 5 is an end view as seen in the direction of an arrow A of Fig. 4.

As shown in Figs. 3 and 4, the folding apparatus 100 is arranged between the cutting rollers 200 for forming the leg-surrounding openings 40, and the holding belt conveyors 500 for holding the web 7 conveyed after being subjected to the folding step S4.

Note that, the web 7 passes through a first conveyance roller R1 and then is fed to the cutting rollers 200. The web 7 having passed through the cutting rollers 200 passes through second to fifth conveyance rollers R2 to R5 and then is fed to the folding apparatus 100. The first to fourth conveyance rollers R1 to R4 rotate about their respective support shafts (not shown).

The folding apparatus 100 includes a large-diameter roller 110 (pressing mechanism), a belt conveyor 120, a folding guide bar 130 (supporting mechanism), a guide mechanism 140, a position detection unit 150 and a position control unit 160.

The large-diameter roller 110 is arranged between the fifth conveyance roller R5 and the guide mechanism 140, and rotates about its support shaft. The large-diameter roller 110 conveys the web 7 while pressing a reference region 11 (first region) of the web 7 toward the belt conveyor 120 and keeping the reference region 11 substantially horizontal. Here, the reference region 11 (Fig. 5) extends from the folding line to the second side edge 20A. The large-diameter roller 110 has a width at least from the folding line to the second side edge 20A.

The belt conveyor 120 is arranged between the large-diameter roller 110 and the holding belt conveyors 500, and is located at the opposite side of the web 7 from the large-diameter roller 110. The belt conveyor 120 conveys the reference region 11 of the web 7 while keeping the reference region 11 substantially horizontal to a contact surface 101 (Fig. 3) of the folding apparatus 100.

The belt conveyor 120 includes: multiple rollers 121; a conveyance belt 122 that is wound and moved around the rollers 121 while supporting the reference region 11 of the web 7; a drive unit (not shown) that causes the conveyance belt 122 to move around the rollers 121; and a suction mechanism 123 that sucks the reference region 11 of the web 7 laid on the conveyance belt 122.

The conveyance belt 122 is driven to move substantially horizontally to the contact surface 101 of the folding apparatus 100. The conveyance belt 122 is preferably moved at a drive speed v1 which is the same as a conveyance speed v2 of the web 7. The conveyance belt 122 includes multiple suction holes 124 (see Figs. 4 and 5) for sucking the reference region 11. Accordingly, the web 7 is conveyed on the conveyance belt 122 while the suction mechanism 123 sucks and holds the reference region 11 on the conveyance belt 122 via the suction holes 124.

The folding guide bar 130 is arranged to extend at least from the vicinity of the fifth conveyance roller R5 to the holding belt conveyors 500, and supports the web 7 at the folding line (i.e., on the center line CL). To put it differently, the folding guide bar 130 partitions the web 7 into the reference region 11 and a folded region 12 (second region) extending from the folding line to the first side edge 10A.

The folding guide bar 130 is arranged to extend in the conveyance direction MD and to be substantially in parallel to the conveyance belt 122. Moreover, the folding guide bar 130 is located on the conveyance belt 122 (see Figs. 3 and 4) .

The folding guide bar 130 includes a slide portion 131 on which the web 7 slides and which has a friction coefficient µ of 0.4 or less. A friction coefficient µ of the slide portion 131 may be between 0.1 and 0.3. A friction coefficient µ of the slide portion 131 may be 0.2.
Specifically, the slide portion 131 forms an outer surface of the folding guide bar 130, and is formed of a tube made of polytetrafluoroethylene (PTFE). Here, the value of the friction coefficient is measured in accordance with the ball-on-disk method defined in JIS R 1613-1993.

Here, the slide portion 131 is not necessarily formed of a tube made of polytetrafluoroethylene, and may be formed of a tube made of, for example, ultra-high molecular polyethylene (having a friction coefficient of 0.2) instead.

The guide mechanism 140 is arranged between the fifth conveyance roller R5 and the holding belt conveyors 500. The guide mechanism 140 guides the first side edge 10A of the web 7 so that the first side edge 10A may be aligned with the second side edge 20A. In other words, the guide mechanism 140 guides the folded region 12 toward the reference region 11.

The guide mechanism 140 includes a pair of first guide rollers 141, a pair of second guide rollers 142, a third guide roller 143, a pair of fourth guide rollers 144, and a fifth guide roller 145. Note that the first to fifth guide rollers 141 to 145 rotate about their respective support shafts.

The first guide rollers 141 raise the folded region 12 of the web 7 with the support of the folding guide bar 130. As shown in Fig. 5, a tilt angle α of the folded region 12 relative to the reference region 11 (fold angle of the crotch portion 30) is gradually reduced as the web 7 passes through the first to fifth guide rollers 141 to 145.

The position detection unit 150 (Fig. 3) is arranged between the fifth guide roller 145 and the holding belt conveyors 500, and detects positions where the first and second side edges 10A and 20A of the web 7 respectively pass. The position detection unit 150 includes a first sensor 151 and a second sensor 152.

The first sensor 151 detects a position where the first side edge 10A passes, whereas the second sensor 152 detects a position where the second side edge 20A passes. The first and second sensors 151 and 152 send position data indicating the respective detected positions to the position control unit 160 (Fig. 3), respectively.

The position control unit 160 is arranged between the fifth guide roller 145 and the holding belt conveyors 500, and includes rollers for adjusting the position of the web 7 in the cross direction CD in response to an instruction from the position detection unit 150.

The holding belt conveyors 500 convey, while holding therebetween the web 7, which has been folded after passing through the folding apparatus 100. The holding belt conveyors 500 at least include: a first holding belt 501 wound and moved around multiple rollers R10; a second holding belt 502 wound and moved around multiple rollers R20; and a drive unit (not shown) causing the first holding belt 501 to move around the rollers R10 and causing the second holding belt 502 to move around the rollers R20. Accordingly, the first and second holding belts 501 and 502 hold the folded web 7 therebetween.

According to the first embodiment having been described thus far, the friction coefficient of the slide portion 131 is 0.4 or less. This helps the web 7, in a portion which includes the folding line and in which the crotch portion 30 is to be formed, to slide more smoothly on the folding guide bar 130 when the folding apparatus 100 folds the web 7 by bringing the first side edge 10A toward the second side edge 20A. This accordingly inhibits the portion to form the crotch portion 30 from being conveyed behind the portions to form the front waistline portion 10 and the back waistline portion 20. Thereby, the first side edge 10A is more likely to be aligned with the second side edge 20A, thus inhibiting a defective absorbent article 1 from being manufactured due to a twist of the crotch portion 30.

If the friction coefficient of the slide portion 131 is more than 0.4 , when coming in contact with the folding guide bar 130, the portion to form the crotch portion 30 may be conveyed behind the portions to form the front waistline portion 10 and the back waistline portion 20 and, thus, may be twisted. Such a twist adversely affects a part ranging from the front waistline portion 10 to the crotch portion 30, and a part ranging from the back waistline portion 20 to the crotch portion 30. For example, the twist displaces the front waistline portion 10 and the back waistline portion 20 from each other in the predetermined regions 50A of the web 7, where the joint portions 50 are to be formed, and accordingly distorts and deforms the entire absorbent article 1 in the joining step S5. This may consequently degrades the appearance of the absorbent article 1 significantly and result in a defective absorbent article 1.

In particular, the web 7 (webs 7A and 7B) is stretchable in the cross direction CD of the web 7, and is asymmetrical with respect to the center line CL. These cause the generation of a force to stretch the first and second side edges 10A and 20A of the web 7 under conveyance in the cross direction CD in which the web 7 is highly stretchable, and thus cause the portion to form the crotch portion 30 to be likely to be conveyed behind the other portions. To cope with this, the friction coefficient of the slide portion 131 is set to 0.4 or less as described above. This inhibits the portion to form the crotch portion 30 from being conveyed behind the other portions.

In the first embodiment, the slide portion 131 is formed of a tube made of or coated with polytetrafluoroethylene (PTFE), which imparts non-adhesive properties to the slide portion 131. This inhibits the portion to form the crotch portion 30 from being conveyed behind the portions to form the front waistline portion 10 and the back waistline portion 20, even if an adhesive used for the bonding of components constituting the absorbent article 1 (leg gather 6B, for example) should leak or exude from the leg-surrounding openings 40.

In the first embodiment, the suction mechanism 123 sucks the reference region 11 of the web 7 laid on the conveyance belt 122. With this configuration, the web 7 is conveyed on the conveyance belt 122 with its reference region 11 sucked and held on the conveyance belt 122. This enables the reference region 11 to be stably conveyed, and thus reliably inhibits the portion to form the crotch portion 30 from being conveyed behind the portions to form the front waistline portion 10 and the back waistline portion 20.

### Second Embodiment

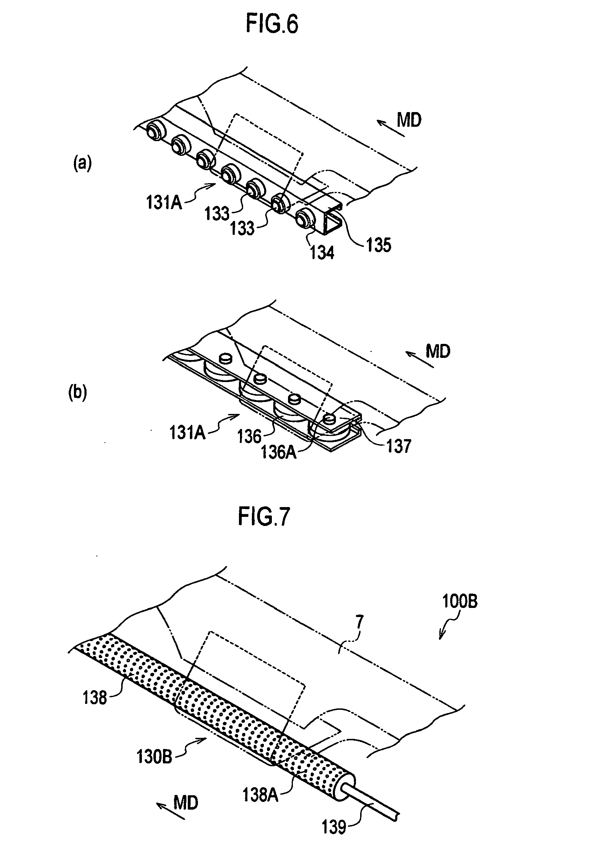

Hereinafter, a configuration of a folding apparatus 100A according to a second embodiment will be explained with reference to Fig. 6, parts (a) and (b) of which are perspective views of various configurations of a folding guide bar 130A according to the second embodiment. Here, description will be provided mainly for differences from the foregoing description, with the same or similar reference signs denoting the same or similar elements.

In the folding apparatus 100 according to the first embodiment, the slide portion 131 of the folding guide bar 130 is formed of a tube made of or coated with polytetrafluoroethylene. By contrast, in the folding apparatus 100A according to the second embodiment, a slide portion 131A of the folding guide bar 130A is formed of a ball conveyor arranged to extend along the folding line.

Specifically, as shown in part (a) of Fig. 6, the slide portion 131A includes: rotatable balls 133; support portions 134 (e.g., sockets) rotatably supporting their respective balls 133; and a bar-shaped fixing portion 135 on which a plurality of the support portions 134 are fixed.

Here, each ball 133 is preferably made of a resin although other arrangements are within the scope of the present invention.

The slide portion 131A is not necessarily a ball conveyor. For example, as shown in part (b) of Fig. 6, the slide portion 131A may be formed of a wheel conveyor including: wheels 136 having their respective shafts 136A which are arranged in a thickness direction of the web 7 being conveyed; and a support portion 137 rotatably supporting the wheels 136.

In the second embodiment described above, the slide portion 131A is formed of a ball or a wheel conveyor. This inhibits the portion to form the crotch portion 30 from being conveyed behind the portions to form the front waistline portion 10 and the back waistline portion 20 when the folding apparatus 100A folds the web 7 by bringing the first side edge 10A toward the second side edge 20A, which is an advantageous effect similar to that of the first embodiment.

### Third Embodiment

Hereinafter, a configuration of a folding apparatus 100B according to a third embodiment will be explained with reference to Fig. 7 which is a perspective view of a part of a folding guide bar 130B according to the third embodiment. Here, description will be provided mainly for differences from the foregoing description, with the same or similar reference signs denoting the same or similar elements.

In the folding apparatus 100 according to the first embodiment, the slide portion 131 of the folding guide bar 130 is formed of a tube made of or coated with polytetrafluoroethylene. By contrast, in the folding apparatus 100B according to the third embodiment, the folding guide bar 130B is formed of a tubular portion 138 and an air supply portion 139.

To be more specific, the tubular portion 138 includes multiple holes 138A. The air supply portion 139 supplies the air to the tubular portion 138. Accordingly, the air supplied by the air supply portion 139 is ejected from the holes 138A after passing through the tubular portion 138.

In the third embodiment described above, the folding guide bar 130B is formed of the tubular portion 138 and the air supply portion 139. This configuration enables the portion, which includes the folding line and in which the crotch portion 30 is to be formed, to be conveyed while being floated from the folding guide bar 130B by the air ejection, and thus reduces friction resistance. This inhibits the portion to form the crotch portion 30 from being conveyed behind the portions to form the front waistline portion 10 and the back waistline portion 20 when the folding apparatus 100B folds the web 7 by bringing the first side edge 10A toward the second side edge 20A.

Note that, the tubular portion 138 does not necessarily include the multiple holes 138A. In order to obtain the same effect as the third embodiment, for example, the tubular portion 138 may include protrusions instead of the holes 138A. It goes without saying that the air supply portion 139 does not need to be provided in this case. In this configuration, the projections of the folding guide bar 130B contact the web 7 in a manner similar to the configuration disclosed with respect to Fig. 6, part (a), except that the projections, unlike the balls 133, do not rotate.

### Further Embodiments

As described above, the details of several embodiments of the present invention have been exemplarily disclosed. It should not be understood that the description and drawings which constitute part of this disclosure limit the present invention. Based on this disclosure, those skilled in the art may easily come up with various alternative embodiments, examples and operation techniques.

For example, the following additional embodiments can be envisaged. Specifically, the absorbent article 1 has been described as including, in combination, the front waistline portion 10, the back waistline portion 20 and the crotch portion 30. The absorbent article 1 is not limited to this configuration, but may be formed entirely as a single unit. In this case, needless to say, a different method of manufacturing an absorbent article is employed.

In addition, the description has been provided for the folding apparatus 100 used in the manufacturing of pants-type disposable diapers. However, the folding apparatus 100 is not limited to such use. For example, the folding apparatus 100 may be used to manufacture various products including open-type disposable diapers, sanitary napkins, panty liners or any other wearing articles. The folding apparatus 100 may be used to fold any web, not necessarily the one to form the front and rear waistline portions and crotch portion as disclosed above.

Further, although PTFE has been described as the material of the slide portion 131 or its coating, any other low-friction materials can be used instead of PTFE. As used herein, a "low friction material" is one that has a friction coefficient of 0.4 or less as measured by the testing standard disclosed herein.

Additionally, the belt conveyor 120 has been described as conveying the reference region 11 of the web 7 while keeping the reference region 11 substantially horizontal to the contact surface 101 of the folding apparatus 100. However, the belt conveyor 120 is not limited to such a configuration. For example, the belt conveyor 120 may convey the reference region 11 while tilting the reference region 11 with respect to the contact surface 101 of the folding apparatus 100, instead. In this case, the conveyance belt 122 is driven to move obliquely with respect to the contact surface 101 of the folding apparatus 100.

Further, the belt conveyor 120 has been described as including the suction mechanism 123. However, the belt conveyor 120 is not limited to such a configuration. Instead, the belt conveyor 120 may include any alternative to the suction mechanism 123, as long as the alternative is capable of attaching and holding the reference region 11 on the conveyance belt 122.

Furthermore, the folding apparatus 100 has been described as including the large-diameter roller 110, the belt conveyor 120, the folding guide bar 130, the guide mechanism 140, and the position control unit 160. However, the folding apparatus 100 is not limited to such a configuration. The folding apparatus 100 may have other configurations as long as it includes at least the large-diameter roller 110, the folding guide bar 130, and the guide mechanism 140 and their equivalents.

For example, the folding apparatus 100 does not necessarily include the belt conveyor 120 as long as the folding apparatus 100 is capable of conveying the web 7. Alternatively, multiple rollers may be installed instead of the belt conveyor 120. Moreover, any number may be selected as the number of conveyance rollers (first to fifth conveyance rollers R1 to R5) or guide rollers (first to fifth guide rollers 141 to 145) .

One or more of the disclosed aspects of the present invention can provide a folding apparatus and a method of manufacturing an absorbent article which are capable of more reliably inhibiting a defective absorbent article from being manufactured due to a twist of a crotch portion including a folding line, in a case where the folding apparatus folds a web to form an absorbent article into two by bringing one side edge of the web toward the opposite side edge thereof.

As described above, the present invention naturally includes various embodiments which are not described herein. Accordingly, the technical scope of the present invention should be determined only by the matters to define the invention in the scope of claims regarded as appropriate based on the description.

The entire content of Japanese Patent Applications 2009-048485 (filed on March 2, 2009) and 2010-041995 (filed on February 26, 2010) are incorporated herein by reference.

### Industrial Applicability

Therefore, according to the present invention, since it is possible to provide a folding apparatus and a method of manufacturing an absorbent article which are capable of more reliably inhibiting a defective absorbent article from being manufactured due to the twist of a crotch portion including a folding position line, in a case where the folding apparatus folds a web to form an absorbent article into two by bringing one side edge of the web toward the opposite side edge thereof, it is useful in manufacturing technology for absorbent articles.

### Reference Signs List

- 1: absorbent article
- 2: top sheet
- 3: back sheet
- 4: absorber
- 5: waterproof sheet
- 6: gather
- 6A: waist gather
- 6B: leg gather
- 7, 7A, 7B: web
- 10: front waistline portion
- 10A: first side edge
- 11: reference region
- 12: folded region
- 20: back waistline portion
- 20A: second side edge
- 30: crotch portion
- 40: leg-surrounding openings
- 50: joint portion
- 50A: predetermined region
- 60: waist openings
- 100, 100A, 100B: folding apparatus
- 101: contact surface
- 110: large-diameter roller (pressing mechanism)
- 120: belt conveyor
- 121: roller
- 122: conveyance belt
- 123: suction mechanism
- 124: suction hole
- 130, 130A: folding guide bar (supporting mechanism)
- 131, 131A: slide portion
- 140: guide mechanism
- 141 to 145: first to fifth guide rollers
- 150: position detection unit
- 151: first sensor
- 152: second sensor
- 160: position control unit
- 200: cutting roller
- 300: holding belt conveyor
- 301: first holding belt
- 302: second holding belt

## Claims

1. A folding apparatus (100) for folding a continuous web (7) into two along a folding line (CL) extending in a conveyance direction of the web by bringing one side edge (10A, 20A) of the web toward an opposite side edge (10A, 20A) thereof, the folding apparatus comprising:
a pressing mechanism (110) configured to press a first region (11) of the web extending from the folding line to the opposite side edge;
a supporting mechanism (130) configured to support the web at the folding line; and
a guide mechanism (140) configured to guide a second region (12) of the web toward the first region, the second region extending from the folding line to the one side edge, wherein
the supporting mechanism includes a slide portion (131A) configured to allow the web to slide thereon, **characterised in that**
the slide portion has a friction coefficient of not more than 0.4, wherein the value of the friction coefficient is measured in accordance with the ball-on-disk method defined in JIS R 1613-1993 and
wherein the slide portion is formed of a ball conveyor (133, 134, 135) arranged to extend along the folding position.

2. A folding apparatus (100B) by which a long web (7) to form an absorbent article is folded into two along a folding position extending in a conveyance direction of the web by bringing one side edge (10A, 20A) of the web toward an opposite side edge (10A, 20A) thereof, the folding apparatus comprising:
a pressing mechanism (110) configured to press a first region (11) extending from the folding position to the opposite side edge;
a supporting mechanism (130B) configured to support the web at the folding position;
and a guide mechanism (140) configured to guide a second region (12) toward the first region, the second region extending from the folding position to the one side edge, wherein
the supporting mechanism includes:
a slide portion configured to allow the web to slide thereon, **characterised in that**
the slide portion has a friction coefficient of not more than 0.4, wherein the value of the friction coefficient is measured in accordance with the ball-on-disk method defined in JIS R 1613-1993; and
the slide portion is formed of a tubular portion (138) including a plurality of holes (138A); and
an air supply portion (139) configured to supply air to the tubular portion (138).

3. The folding apparatus according to claim 1 or claim 2, further comprising
a belt conveyor (120) arranged at an opposite side of the web from the pressing mechanism, wherein
the belt conveyor includes:
a conveyance belt (122) configured to be wound and moved around a plurality of rollers (121) while supporting the first region;
and a suction mechanism (123) configured to suck the first region laid on the conveyance belt

4. A method of manufacturing an absorbent article (1) having:
a front waistline portion (10) to be fitted to a front waist of a wearer; a back waistline portion (20) to be fitted to a back waist of the wearer; a crotch portion (30) to be fitted to a crotch of the wearer; and leg-surrounding opening regions (40) open at both sides of the crotch portion, the method comprising the steps of:
forming the leg- surrounding opening regions in a long web (7) to form the absorbent article; and
causing a folding apparatus (100) to fold the web into two along a folding position extending in a conveyance direction of the web by bringing one side edge (10A, 20A) of the web toward an opposite side edge (10A, 20A) thereof, wherein
the folding apparatus includes:
a pressing mechanism (110) configured to press a first region (11) extending from the folding position to the opposite side edge;
a supporting mechanism (130) configured to support the web at the folding position; and
a guide mechanism (140) configured to guide a second region (12) toward the first region, the second region extending from the folding position to the one side edge,
the supporting mechanism includes a slide portion (131A) configured to allow the web to slide thereon, and the slide portion has a friction coefficient of not more than 0.4, wherein the value of the friction coefficient is measured in accordance with the ball-on-disk method defined in JIS R 1613-1993 and
wherein the slide portion is formed of a ball conveyor (133, 134, 135) arranged to extend along the folding position.

5. A method of manufacturing an absorbent article (1) having:
a front waistline portion (10) to be fitted to a front waist of a wearer; a back waistline portion (20) to be fitted to a back waist of the wearer; a crotch portion (30) to be fitted to a crotch of the wearer; and leg-surrounding opening regions (40) open at both sides of the crotch portion, the method comprising the steps of:
forming the leg- surrounding opening regions in a long web (7) to form the absorbent article; and
causing a folding apparatus (100B) to fold the web into two along a folding position extending in a conveyance direction of the web by bringing one side edge (10A, 20A) of the web toward an opposite side edge (10A, 20A) thereof, wherein
the folding apparatus includes:
a pressing mechanism (110) configured to press a first region extending from the folding position to the opposite side edge;
a supporting mechanism (130B) configured to support the web at the folding position; and
a guide mechanism (140) configured to guide a second region toward the first region, the second region extending from the folding position to the one side edge,
the supporting mechanism includes a slide portion configured to allow the web to slide thereon, and the slide portion has a friction coefficient of not more than 0.4, wherein the value of the friction coefficient is measured in accordance with the ball-on-disk method defined in JIS R 1613-1993; and
the slide portion is formed of a tubular portion (138) including a plurality of holes (138A); and
an air supply portion (139) configured to supply air to the tubular portion.

6. The method of manufacturing an absorbent article according to claim 4 or claim 5, wherein the web is asymmetrical with respect to a center line (CL) extending in the conveyance direction of the web while passing through a center in a cross direction perpendicular to the conveyance direction of the web.

7. The method of manufacturing an absorbent article according to any one of claims 4 to 6, wherein the web is stretchable in the cross direction of the web.

## Patentansprüche

1. Eine Faltvorrichtung (100) zum Falten einer Endlosbahn (7) in halb entlang einer Faltlinie (CL), die sich in einer Beförderungsrichtung der Bahn erstreckt, indem eine Seitenkante (10A, 20A) der Bahn zu einer gegenüberliegenden Seitenkante (10A, 20A) davon hin gebracht wird, wobei die Faltvorrichtung Folgendes beinhaltet:
einen Druckmechanismus (110), der konfiguriert ist, um eine erste Region (11) der Bahn, die sich von der Faltlinie zu der gegenüberliegenden Seitenkante erstreckt, zu drücken;
einen Stützmechanismus (130), der konfiguriert ist, um die Bahn an der Faltlinie zu stützen; und
einen Führungsmechanismus (140), der konfiguriert ist, um eine weite Region (12) der Bahn zur ersten Region hin zu führen, wobei sich die weite Region von der Faltlinie zu der einen Seitenkante erstreckt, wobei
der Stützmechanismus einen Gleitabschnitt (131A) umfasst, der konfiguriert ist, um zu ermöglichen, dass die Bahn darauf gleitet, **dadurch gekennzeichnet, dass**
der Gleitabschnitt einen Reibungskoeffizienten von nicht mehr als 0,4 aufweist, wobei der Wert des Reibungskoeffizienten gemäß dem in JIS R 1613-1993 definierten Kugel-Scheibe-Verfahren gemessen wird, und
wobei der Gleitabschnitt aus einem Kugelförderer (133, 134, 135) gebildet ist, der eingerichtet ist, um sich entlang der Faltposition zu erstrecken.

2. Eine Faltvorrichtung (100B), durch die eine lange Bahn (7) zum Bilden eines absorptionsfähigen Artikels entlang einer Faltposition, die sich in einer Beförderungsrichtung der Bahn erstreckt, in halb gefaltet wird, indem eine Seitenkante (10A, 20A) der Bahn zu einer gegenüberliegenden Seitenkante (10A, 20A) davon hin gebracht wird, wobei die Faltvorrichtung Folgendes beinhaltet:
einen Druckmechanismus (110), der konfiguriert ist, um eine erste Region (11), die sich von der Faltposition zu der gegenüberliegenden Seitenkante erstreckt, zu drücken;
einen Stützmechanismus (130B), der konfiguriert ist, um die Bahn an der Faltposition zu stützen;
und einen Führungsmechanismus (140), der konfiguriert ist, um eine weite Region (12) zur ersten Region hin zu führen, wobei sich die zweite Region von der Faltposition zu der einen Seitenkante erstreckt, wobei
der Stützmechanismus Folgendes umfasst:
einen Gleitabschnitt, der konfiguriert ist, um zu ermöglichen, dass die Bahn darauf gleitet, **dadurch gekennzeichnet, dass**
der Gleitabschnitt einen Reibungskoeffizienten von nicht mehr als 0,4 aufweist, wobei der Wert des Reibungskoeffizienten gemäß dem in JIS R 1613-1993 definierten Kugel-Scheibe-Verfahren gemessen wird; und
der Gleitabschnitt aus einem röhrenförmigen Abschnitt (138) gebildet ist, der eine Vielzahl von Löchern (138A) umfasst; und
einen Luftzufuhrabschnitt (139), der konfiguriert ist, um dem röhrenförmigen Abschnitt (138) Luft zuzuführen.

3. Faltvorrichtung gemäß Anspruch 1 oder Anspruch 2, die ferner Folgendes beinhaltet:
einen Bandförderer (120), der auf einer dem Druckmechanismus gegenüberliegenden Seite der Bahn eingerichtet ist, wobei
der Bandförderer Folgendes umfasst:
ein Beförderungsband (122), das konfiguriert ist, um um eine Vielzahl von Walzen (121) gewunden und bewegt zu werden, während es die erste Region stützt;
und einen Saugmechanismus (123), der konfiguriert ist, um die auf das Beförderungsband gelegte erste Region anzusaugen.

4. Ein Verfahren zum Herstellen eines absorptionsfähigen Artikels (1), der Folgendes aufweist:
einen vorderen Taillenabschnitt (10), der an eine vordere Taille eines Trägers gepasst werden soll; einen hinteren Taillenabschnitt (20), der an eine hintere Taille des Trägers gepasst werden soll; einen Schrittabschnitt (30), der an einen Schritt des Trägers gepasst werden soll; und beinumgebende Öffnungsregionen (40), die an beiden Seiten des Schrittabschnitts offen sind, wobei das Verfahren die folgenden Einzelschritte beinhaltet:
Bilden der beinumgebenden Öffnungsregionen in einer langen Bahn (7), um den absorptionsfähigen Artikel zu bilden; und
Bewirken, dass eine Faltvorrichtung (100) die Bahn entlang einer Faltposition, die sich in einer Beförderungsrichtung der Bahn erstreckt, in halb faltet, indem eine Seitenkante (10A, 20A) der Bahn zu einer gegenüberliegenden Seitenkante (10A, 20A) davon hin gebracht wird, wobei
die Faltvorrichtung Folgendes umfasst:
einen Druckmechanismus (110), der konfiguriert ist, um eine erste Region (11), die sich von der Faltposition zu der gegenüberliegenden Seitenkante erstreckt, zu drücken;
einen Stützmechanismus (130), der konfiguriert ist, um die Bahn an der Faltposition zu stützen; und
einen Führungsmechanismus (140), der konfiguriert ist, um eine weite Region (12) zur ersten Region hin zu führen, wobei sich die weite Region von der Faltposition zu der einen Seitenkante erstreckt,
der Stützmechanismus einen Gleitabschnitt (131A) umfasst, der konfiguriert ist, um zu ermöglichen, dass die Bahn darauf gleitet, und der Gleitabschnitt einen Reibungskoeffizienten von nicht mehr als 0,4 aufweist, wobei der Wert des Reibungskoeffizienten gemäß dem in JIS R 1613-1993 definierten Kugel-Scheibe-Verfahren gemessen wird; und
wobei der Gleitabschnitt aus einem Kugelförderer (133, 134, 135) gebildet ist, der eingerichtet ist, um sich entlang der Faltposition zu erstrecken.

5. Ein Verfahren zum Herstellen eines absorptionsfähigen Artikels (1), der Folgendes aufweist:
einen vorderen Taillenabschnitt (10), der an eine vordere Taille eines Trägers gepasst werden soll; einen hinteren Taillenabschnitt (20), der an eine hintere Taille des Trägers gepasst werden soll; einen Schrittabschnitt (30), der an einen Schritt des Trägers gepasst werden soll; und beinumgebende Öffnungsregionen (40), die an beiden Seiten des Schrittabschnitts offen sind, wobei das Verfahren die folgenden Einzelschritte beinhaltet:
Bilden der beinumgebenden Öffnungsregionen in einer langen Bahn (7), um den absorptionsfähigen Artikel zu bilden; und
Bewirken, dass eine Faltvorrichtung (100B) die Bahn entlang einer Faltposition, die sich in einer Beförderungsrichtung der Bahn erstreckt, in halb faltet, indem eine Seitenkante (10A, 20A) der Bahn zu einer gegenüberliegenden Seitenkante (10A, 20A) davon hin gebracht wird, wobei
die Faltvorrichtung Folgendes umfasst:
einen Druckmechanismus (110), der konfiguriert ist, um eine erste Region, die sich von der Faltposition zu der gegenüberliegenden Seitenkante erstreckt, zu drücken;
einen Stützmechanismus (130B), der konfiguriert ist, um die Bahn an der Faltposition zu stützen; und
einen Führungsmechanismus (140), der konfiguriert ist, um eine weite Region zur ersten Region hin zu führen, wobei sich die weite Region von der Faltposition zu der einen Seitenkante erstreckt,
der Stützmechanismus einen Gleitabschnitt umfasst, der konfiguriert ist, um zu ermöglichen, dass die Bahn darauf gleitet, und der Gleitabschnitt einen Reibungskoeffizienten von nicht mehr als 0,4 aufweist, wobei der Wert des Reibungskoeffizienten gemäß dem in JIS R 1613-1993 definierten Kugel-Scheibe-Verfahren gemessen wird; und
der Gleitabschnitt aus einem röhrenförmigen Abschnitt (138) gebildet ist, der eine Vielzahl von Löchern (138A) umfasst; und
einen Luftzufuhrabschnitt (139), der konfiguriert ist, um dem röhrenförmigen Abschnitt Luft zuzuführen.

6. Verfahren zum Herstellen eines absorptionsfähigen Artikels gemäß Anspruch 4 oder Anspruch 5, wobei die Bahn asymmetrisch ist in Hinsicht auf eine Mittellinie (CL), die sich in der Beförderungsrichtung der Bahn erstreckt, während sie durch eine Mitte in einer Querrichtung geht, die zu der Beförderungsrichtung der Bahn senkrecht ist.

7. Verfahren zum Herstellen eines absorptionsfähigen Artikels gemäß einem der Ansprüche 4 bis 6, wobei die Bahn in der Querrichtung der Bahn dehnbar ist.

## Revendications

1. Un appareil de pliage (100) destiné à plier une bande continue (7) en deux le long d'une ligne de pliage (CL) qui s'étend dans un sens de transport de la bande en ramenant un premier bord latéral (10A, 20A) de la bande vers un bord latéral opposé (10A, 20A) de celle-ci, l'appareil de pliage comprenant :
un mécanisme de pression (110) configuré pour exercer une pression sur une première région (11) de la bande qui s'étend de la ligne de pliage au bord latéral opposé ;
un mécanisme de support (130) configuré pour supporter la bande au niveau de la ligne de pliage ; et
un mécanisme de guidage (140) configuré pour guider une deuxième région (12) de la bande vers la première région, la deuxième région s'étendant de la ligne de pliage au premier bord latéral, dans lequel
le mécanisme de support comporte une portion de glissement (131A) configurée pour permettre à la bande de glisser dessus, **caractérisé en ce que**
la portion de glissement a un coefficient de frottement qui ne dépasse pas 0,4, la valeur du coefficient de frottement étant mesurée conformément à la méthode bille sur disque définie dans JIS R 1613-1993 et
dans lequel la portion de glissement est formée d'un transporteur à bille (133, 134, 135) arrangé pour s'étendre le long de la position de pliage.

2. Un appareil de pliage (100B) grâce auquel une longue bande (7) devant former un article absorbant est pliée en deux le long d'une position de pliage qui s'étend dans un sens de transport de la bande en ramenant un premier bord latéral (10A, 20A) de la bande vers un bord latéral opposé (10A, 20A) de celle-ci, l'appareil de pliage comprenant :
un mécanisme de pression (110) configuré pour exercer une pression sur une première région (11) qui s'étend de la position de pliage au bord latéral opposé ;
un mécanisme de support (130B) configuré pour supporter la bande au niveau de la position de pliage ;
et un mécanisme de guidage (140) configuré pour guider une deuxième région (12) vers la première région, la deuxième région s'étendant de la position de pliage au premier bord latéral, dans lequel
le mécanisme de support comporte :
une portion de glissement configurée pour permettre à la bande de glisser dessus,
**caractérisé en ce que**
la portion de glissement a un coefficient de frottement qui ne dépasse pas 0,4, la valeur du coefficient de frottement étant mesurée conformément à la méthode bille sur disque définie dans JIS R 1613-1993 ; et
la portion de glissement est formée d'une portion tubulaire (138) comportant une pluralité de trous (138A) ; et
une portion d'alimentation en air (139) configurée pour alimenter en air la portion tubulaire (138).

3. L'appareil de pliage selon la revendication 1 ou la revendication 2, comprenant en outre un transporteur à courroie (120) arrangé au niveau d'un côté opposé de la bande par rapport au mécanisme de pression, dans lequel
le transporteur à courroie comporte :
une courroie de transport (122) configurée pour être amenée à s'enrouler et à se déplacer autour d'une pluralité de rouleaux (121) tout en supportant la première région ;
et un mécanisme de succion (123) configuré pour exercer une succion sur la première région posée sur la courroie de transport.

4. Une méthode de fabrication d'un article absorbant (1) ayant :
une portion de taille avant (10) devant venir se mettre sur une taille avant d'une personne portant l'article ; une portion de taille arrière (20) devant venir se mettre sur une taille arrière de la personne portant l'article ; une portion d'entrejambe (30) devant venir se mettre sur une entrejambe de la personne portant l'article ; et des régions d'ouverture entourant les jambes (40) ouvertes au niveau des deux côtés de la portion d'entrejambe, la méthode comprenant les étapes consistant à :
former les régions d'ouverture entourant les jambes dans une longue bande (7) devant former l'article absorbant ; et
amener un appareil de pliage (100) à plier la bande en deux le long d'une position de pliage qui s'étend dans un sens de transport de la bande en ramenant un premier bord latéral (10A, 20A) de la bande vers un bord latéral opposé (10A, 20A) de celle-ci, dans laquelle
l'appareil de pliage comporte :
un mécanisme de pression (110) configuré pour exercer une pression sur une première région (11) qui s'étend de la position de pliage au bord latéral opposé ;
un mécanisme de support (130) configuré pour supporter la bande au niveau de la position de pliage ; et
un mécanisme de guidage (140) configuré pour guider une deuxième région (12) vers la première région, la deuxième région s'étendant de la position de pliage au premier bord latéral,
le mécanisme de support comporte une portion de glissement (131A) configurée pour permettre à la bande de glisser dessus, et la portion de glissement a un coefficient de frottement qui ne dépasse pas 0,4, la valeur du coefficient de frottement étant mesurée conformément à la méthode bille sur disque définie dans JIS R 1613-1993 et
dans laquelle la portion de glissement est formée d'un transporteur à bille (133, 134, 135) arrangé pour s'étendre le long de la position de pliage.

5. Une méthode de fabrication d'un article absorbant (1) ayant :
une portion de taille avant (10) devant venir se mettre sur une taille avant d'une personne portant l'article ; une portion de taille arrière (20) devant venir se mettre sur une taille arrière de la personne portant l'article ; une portion d'entrejambe (30) devant venir se mettre sur une entrejambe de la personne portant l'article ; et des régions d'ouverture entourant les jambes (40) ouvertes au niveau des deux côtés de la portion d'entrejambe, la méthode comprenant les étapes consistant à :
former les régions d'ouverture entourant les jambes dans une longue bande (7) devant former l'article absorbant ; et
amener un appareil de pliage (100B) à plier la bande en deux le long d'une position de pliage qui s'étend dans un sens de transport de la bande en ramenant un premier bord latéral (10A, 20A) de la bande vers un bord latéral opposé (10A, 20A) de celle-ci, dans laquelle
l'appareil de pliage comporte :
un mécanisme de pression (110) configuré pour exercer une pression sur une première région qui s'étend de la position de pliage au bord latéral opposé ;
un mécanisme de support (130B) configuré pour supporter la bande au niveau de la position de pliage ; et
un mécanisme de guidage (140) configuré pour guider une deuxième région vers la première région, la deuxième région s'étendant de la position de pliage au premier bord latéral,
le mécanisme de support comporte une portion de glissement configurée pour permettre à la bande de glisser dessus, et la portion de glissement a un coefficient de frottement qui ne dépasse pas 0,4, la valeur du coefficient de frottement étant mesurée conformément à la méthode bille sur disque définie dans JIS R 1613-1993 ; et
la portion de glissement est formée d'une portion tubulaire (138) comportant une pluralité de trous (138A) ; et
une portion d'alimentation en air (139) configurée pour alimenter en air la portion tubulaire.

6. La méthode de fabrication d'un article absorbant selon la revendication 4 ou la revendication 5, dans laquelle la bande est asymétrique par rapport à une ligne centrale (CL) qui s'étend dans le sens de transport de la bande tout en passant par un centre dans un sens travers perpendiculaire au sens de transport de la bande.

7. La méthode de fabrication d'un article absorbant selon l'une quelconque des revendications 4 à 6, dans laquelle la bande est étirable dans le sens travers de la bande.
